# EUROPEAN PATENT SPECIFICATION

(11) **EP 4 370 066 B1**
(45) Date of publication and mention of the grant of the patent: **20.05.2026**
(21) Application number: 22753831.1
(22) Date of filing: 13.07.2022
(51) Int. Cl.: A61F 2/24, A61B 17/122

(54) **MITRAL VALVE LEAFLET CLIP**
MITRALKLAPPENSEGELKLAMMER
CLIP DE FEUILLET DE VALVE MITRALE

(30) Priority: 14.07.2021 US 202163221529 P
(43) Date of publication of application: 22.05.2024
(73) Proprietor: Boston Scientific Scimed Inc., Maple Grove, Minnesota 55311 (US)
(72) Inventor: GIESE, Troy Anthony, Blaine, Minnesota 55449 (US); FREKING, Michael Mathias, Arden Hills, Minnesota 55112 (US); KILLEEN, Larry Michael, Elk River, Minnesota 55330 (US)
(74) Representative: Peterreins Schley
(86) International application number: PCT/US2022/036959
(87) International publication number: WO 2023/287880

(56) References cited:
- WO-A1-2018/013856
- WO-A1-2019/139904
- US-A1- 2018 021 133
- US-A1- 2019 015 208
- US-A1- 2021 169 651

## Description

### Technical Field

The disclosure pertains to medical devices and more particularly to devices for heart valve repair, and methods for using such medical devices.

### Background

A wide variety of medical devices have been developed for medical use including, for example, medical devices utilized to repair heart valves. These medical devices may be used to treat valves of the heart and venous valves, and are manufactured and used according to any one of a variety of different methods. Of the known medical devices and methods, each has certain advantages and disadvantages. There is an ongoing need to provide alternative medical devices as well as alternative methods for manufacturing and using the medical devices.

US 2021/0169651 A1 discloses a valve fixation device with an elongate member biased towards a closed configuration. WO 2018/013856 A1 discloses a clip for immobilizing leaflets of a valve. US 2019/015208 A1 discloses a valve repair device with first and second clasps.

### Summary

This disclosure provides design, material, manufacturing method, and use alternatives for medical devices. The present invention is directed to a fixation device for engaging heart valve leaflets as set forth in the appended claims. In accordance with the invention, the fixation device for engaging heart valve leaflets comprises a center member with a first end, an opposite second end, and a length extending therebetween, first and second arms each having a first free end and an opposite second end, the second end of each arm extending from the second end of the center member, the first and second arms biased in a first position adjacent the center member and moveable to a second position spaced apart from the center member. The fixation device is configured to capture a first leaflet between a first portion of the first arm and the center member and a second leaflet between a first portion of the second arm and the center member when returning from the second position to the first position. The fixation device comprises an actuator configured to move the first and second arms from the first position to the second position. The fixation device comprises an actuator configured to move the first and second arms from the first position to the second position.

The actuator is removable and includes a first wire removably connected to the first arm and a second wire removably connected to the second arm. The first arm has an opening configured to receive a loop in the first wire and the second arm has an opening configured to receive a loop in the second wire. Opposing free ends of each wire extend through a lumen in the center member, wherein a pulling force applied on the free ends moves the first and second arms from the biased first position to the second position, wherein releasing the pulling force allows the first and second arms to return to the first position.

The above summary of some embodiments, aspects, and/or examples is not intended to describe each embodiment or every implementation of the present disclosure. The figures and the detailed description which follows more particularly exemplify these embodiments.

### Brief Description of the Drawings

The disclosure may be more completely understood in consideration of the following detailed description of various embodiments in connection with the accompanying drawings, in which:
FIG. 1 is a partial cut-away view of an example heart having a "normal" atrial valve;
FIG. 2 is a partial cut-away view of an example heart having an "abnormal" atrial valve;
FIG. 3 is a perspective view of an example heart valve fixation device;
FIG. 4 is a front view of the example fixation device of FIG. 3 in a closed position;
FIG. 5 is a front view of the example fixation device of FIG. 3 in an open position;
FIG. 6 is a partial cut-away view of the example fixation device of FIG. 3 implanted in a heart valve;
FIG. 7 is a partial top view of the heart valve of FIG. 6 with the fixation device;
FIG. 8 is a perspective view of another example heart valve fixation device;
FIG. 9 is a front view of the example fixation device of FIG. 8 in a closed position; and
FIG. 10 is a front view of the example fixation device of FIG. 8 in an open position.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications falling within the scope of the disclosure.

### Detailed Description

The present invention is directed to a fixation device for engaging heart valve leaflets as set forth in the appended claims. For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5). Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the disclosure are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "withdraw", variants thereof, and the like, may be generally considered with respect to the positioning, direction, and/or operation of various elements relative to a user/operator/manipulator of the device, wherein "proximal" and "withdraw" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device.

The term "extent" may be understood to mean a greatest measurement of a stated or identified dimension, unless the extent or dimension in question is preceded by or identified as a "minimum", which may be understood to mean a smallest measurement of the stated or identified dimension. For example, "outer extent" may be understood to mean a maximum outer dimension, "radial extent" may be understood to mean a maximum radial dimension, "longitudinal extent" may be understood to mean a maximum longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage, while a "minimum extent" may be considered a smallest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

The terms "monolithic" and "unitary" shall generally refer to an element or elements made from or consisting of a single structure or base unit/element. A monolithic and/or unitary element shall exclude structure and/or features made by assembling or otherwise joining multiple discrete elements together.

It is noted that references in the specification to "an embodiment", "some embodiments", "other embodiments", etc., indicate that the embodiment(s) described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it would be within the knowledge of one skilled in the art to effect the particular feature, structure, or characteristic in connection with other embodiments, whether or not explicitly described, unless clearly stated to the contrary. That is, the various individual elements described below, even if not explicitly shown in a particular combination, are nevertheless contemplated as being combinable or arrangeable with each other to form other additional embodiments or to complement and/or enrich the described embodiment(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previously-used numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

The following description should be read with reference to the drawings, which are not necessarily to scale, wherein similar elements in different drawings are numbered the same. The detailed description and drawings are intended to illustrate but not limit the disclosure. Those skilled in the art will recognize that the various elements described and/or shown may be arranged in various combinations and configurations without departing from the scope of the disclosure. The detailed description and drawings illustrate example embodiments of the disclosure. However, in the interest of clarity and ease of understanding, while every feature and/or element may not be shown in each drawing, the feature(s) and/or element(s) may be understood to be present regardless, unless otherwise specified.

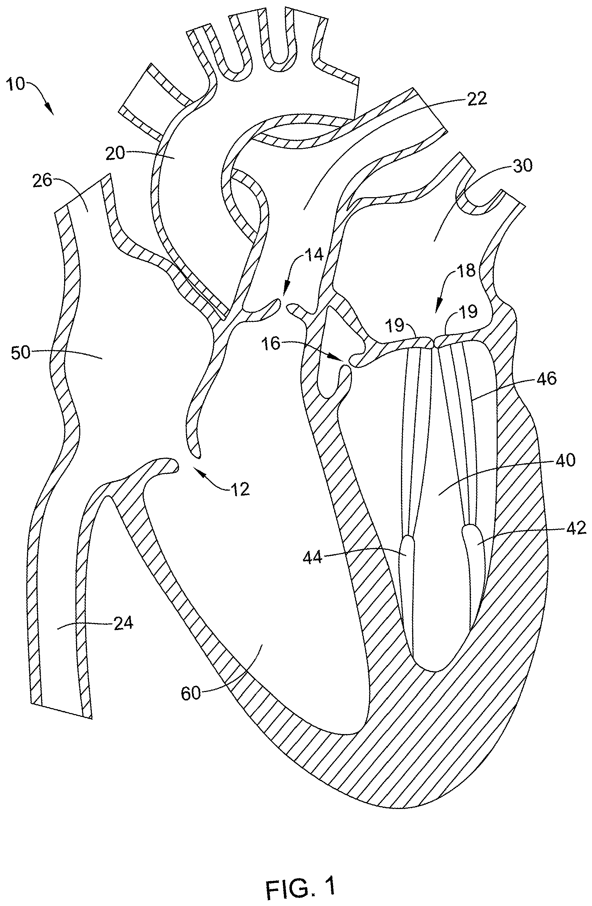

Diseases and/or medical conditions that impact the cardiovascular system are prevalent throughout the world. Some mammalian hearts (e.g., human, etc.) include four heart valves: a tricuspid valve 12, a pulmonary valve 14, an aortic valve 16, and a mitral valve 18, as seen in an example heart 10 illustrated in FIG. 1. The purpose of the heart valves is to control blood flow into the heart 10 from the inferior vena cava 24 and/or the superior vena cava 26, through the heart 10, and out of the heart 10 into the major blood vessels connected to the heart 10, such as the aorta 20, the pulmonary artery 22, for example. Each heart valve may have a plurality of valve leaflets configured to shift between an open configuration permitting fluid flow through the heart valve in an antegrade direction, and a closed configuration wherein free edges of the valve leaflets coapt to substantially prevent fluid flow through the heart valve in a retrograde direction. The heart 10 may also include a left atrium 30, a left ventricle 40, a right atrium 50, and a right ventricle 60. The left ventricle 40 may include a first papillary muscle 42 attached to and/or extending from a wall of the left ventricle 40, a second papillary muscle 44 attached to and/or extending from the wall of the left ventricle 40, and a plurality of chordae 46 connecting the first papillary muscle 42 and the second papillary muscle 44 to the leaflets 19 of the mitral valve 18. In a normally functioning heart valve, blood is permitted to pass or flow downstream through the heart valve (e.g., from an atrium to a ventricle, from a ventricle to an artery, etc.) when the heart valve is open (e.g., during diastole), and when the heart valve is closed (e.g., during systole), blood is prevented from passing or flowing back upstream through the heart valve (e.g., from a ventricle to an atrium, etc.).

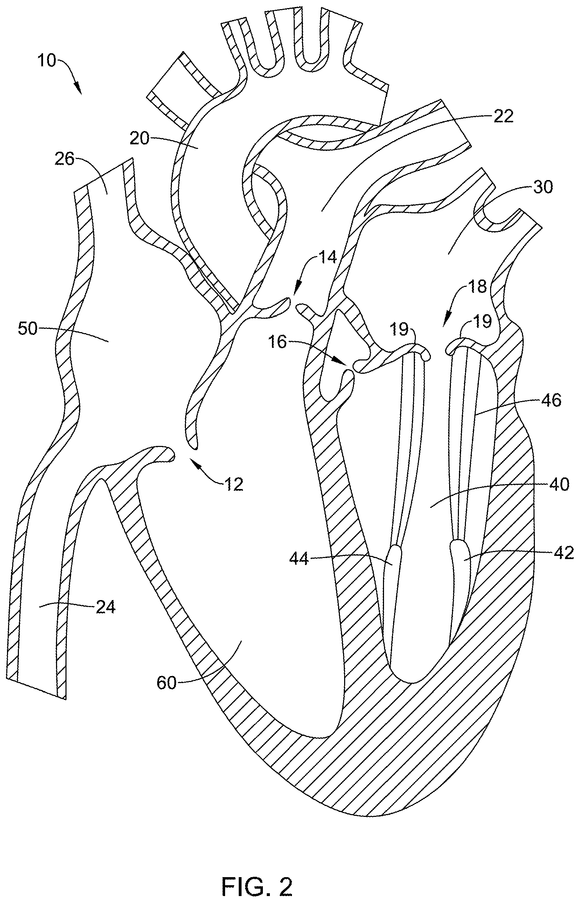

Roughly 1.6 to 2.8 million people in the U.S. have atrioventricular heart valve malfunctions that impede cardiac output, creating lower quality of life and lifespan. As heart disease progresses, the chordae tendineae that connect the papillary muscles to the leaflet begin to stretch and rupture. In some instances, when regurgitation (e.g., mitral regurgitation) occurs, a heart valve (e.g., the mitral valve 18) fails to open and/or close properly such that blood is permitted to pass or flow back upstream through the heart valve (e.g., from a ventricle to an atrium, etc.). In some cases, the defective heart valve may have leaflets 19 that may not close, or may not be capable of closing, completely, as seen in FIG. 2.

Regurgitation prevents an adequate supply of blood to be delivered through the cardiovascular systems. Conventional open heart surgery solutions include replacing stretched and ruptured chordae by sewing an expanded polytetrafluoroethylene (ePTFE) suture to the papillary and the leaflet in order to mimic the natural chordae. However, open heart surgery carries a significant morbidity rate, risk of complications, and disability during recovery. Minimally invasive solutions include transcatheter artificial valve replacement, but can carry lifelong treatment with anticoagulants.

Disclosed herein are apparatus, medical devices, and/or methods that may be used to diagnose, treat, and/or repair a portion of the cardiovascular system. The disclosed mitral regurgitation treatment method(s) and associated medical device(s) may be performed/used percutaneously via minimally-invasive intravascular techniques, or in an alternative method, using open-heart surgical methods. The device(s) and method(s) disclosed herein may also provide a number of additional desirable features and/or benefits as described in more detail below. For the purpose of this disclosure, the discussion below is directed toward repairing the mitral valve 18 and will be so described in the interest of brevity. This, however, is not intended to be limiting as the skilled person will recognize that the following discussion may also apply to the aortic valve or another heart valve with no or minimal changes to the structure and/or scope of the disclosure.

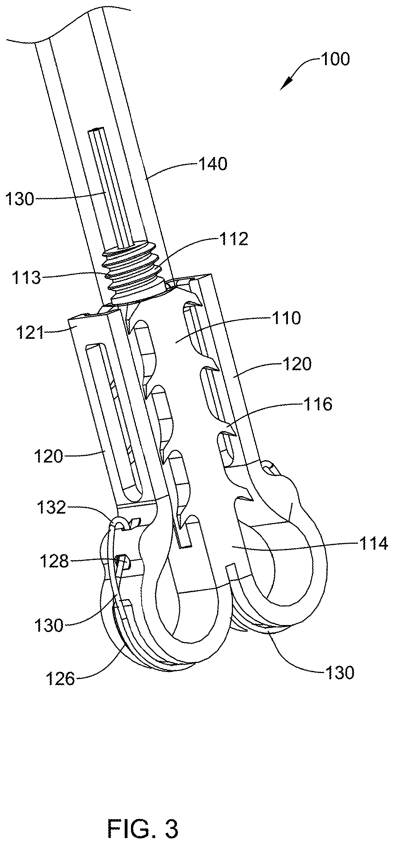

In accordance with the invention and as will be described in greater detail below, FIG. 3 illustrates an example edge-to-edge fixation device 100 in the form of a leaflet clip configured for minimally invasive implantation in a beating heart to repair mitral regurgitation. Specifically, FIG. 3 illustrates a wire actuated fixation device 100 that includes a center member 110, first and second arms 120, and first and second actuators. In the example shown in FIG. 3, the actuators are first and second wires 130. In other examples, the actuators may be braided sutures or cables. The center member 110 has a first end 112 and a second end 114. The first end 112 may include a connection element 113 for removable engagement with a catheter 140. In some examples, the connection element 113 may include threading that matches threading on the distal end of a delivery catheter 140. The first and second arms 120 extend from the second end 114 of the center member 110 and have a free end 121. The center member 110 may include a surface texture configured to retain the valve leaflets when the fixation device 100 is engaged with opposing valve leaflets. In some examples, the surface texture includes a plurality of radially extending projections 116. The projections 116 may be sharpened. In other examples, the surface of the center member 110 may be roughened or textured.

In some examples, the center member 110 and the first and second arms 120 are formed as a monolithic structure. In other examples, the first and second arms 120 may be formed separately and attached to the center member 110. The arms 120 may be made of a material that is more flexible than the center member 110.

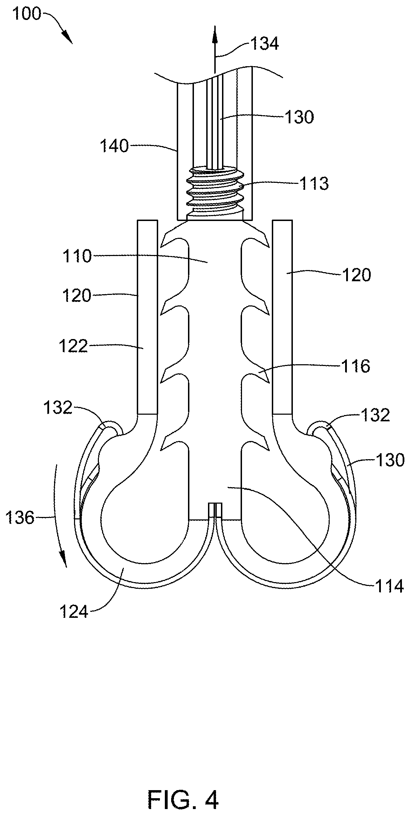

In the example shown in FIGS. 3 and 4, the first and second wires 130 extend through a lumen extending the length of the center member 110. A first end of each wire 130 may exit the lumen at the second end 114 of the center member 110, and then travel along a groove or channel 126 in each arm 120. The wire 130 may be threaded through an opening 128 in the arm 120, forming a loop 132 and then return along the channel 126 and lumen and through the catheter 140 such that free ends of each wire 130 reside proximal of the catheter 140.

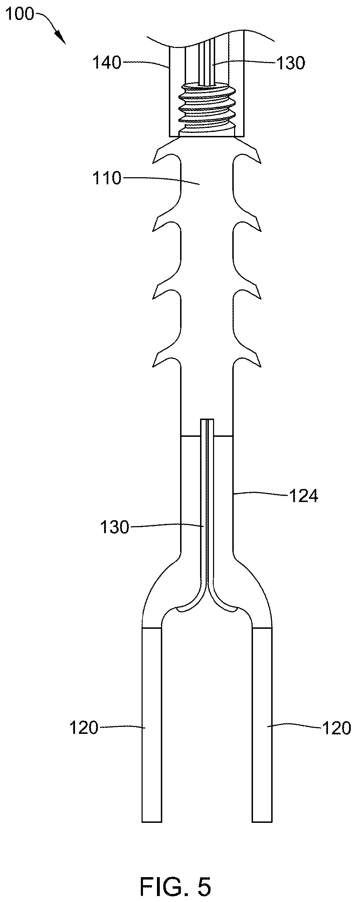

At least a portion of each of the arms 120 is flexible and configured to move between a first, closed position as shown in FIG. 4, and a second, fully open position as shown in FIG. 5. In the first position, at least a first portion 122 of each of the arms 120 is closely adjacent the projections 116. In some examples, the first portion 122 of each arm 120 may be in direct contact with the projections 116. The free ends of the projections 116 may be blunt, tapered or sharpened, to aid in securing the fixation device 100 to the valve leaflets 19. Each arm 120 may have the same flexibility along its entire length. In other examples, the first portion 122 of each arm 120 may be rigid, and the second portion 124 of each arm 120 may be flexible, allowing the arms 120 to be moved into the second position. The arms 120 may be biased in the first position, in which the arms 120 are substantially parallel to the center member 110, at a 0-degree angular position relative to a longitudinal axis of the center member 110. In accordance with the invention, the arms 120 can be actuated by pulling on the ends of the wires 130. An upward pulling force on the ends of the wires 130, indicated by arrow 134, will translate into a downward force, indicated by arrow 136, as the loops 132 engage the openings 128 in the arms 120. Continued upward pulling on the wires 130 will move the arms 120 through a range of positions from 0 degrees through 180 degrees in the second, fully open position, as shown in FIG. 5. The arms 120 may be moved to any angular position between 0 degrees and 180 degrees. The arms 120 may be actuated simultaneously by pulling on all four free ends of the two wires 130 at the same time. Each arm 120 may also be actuated separately by pulling on the two free ends of the wire 130 connected to that arm.

The fixation device 100 attached to the catheter 140 may be inserted through the vasculature and into the heart while in the first position. This provides the advantage of allowing the wires 130 to be relaxed during delivery. In other examples, the fixation device 100 may be delivered while in the second position, with the wires 130 held under tension to keep the arms 120 in the second position, as shown in FIG. 5. Once positioned within the valve to be treated, the fixation device 100 may be secured to both valve leaflets 19 at the same time by actuating both arms 120 simultaneously, or the leaflets 19 may be secured one at a time by actuating the arms 120 sequentially. Securing the leaflets one at a time may be easier, particularly when the heart is beating and the leaflets are moving. The flexibility of the fixation device 100 being secured to the leaflets at the same time or one at a time provides the advantage of allowing the operator to choose the implantation process that is desired for the particular patient's valve anatomy.

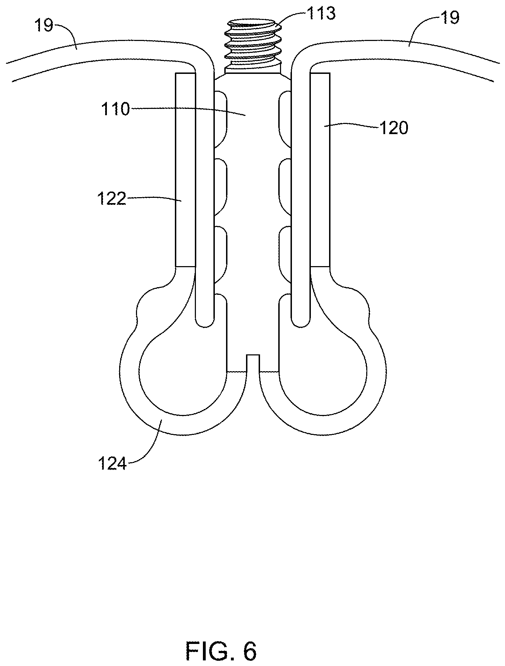

Once the fixation device 100, in the first position, is disposed within the valve, the wire 130 on a first arm 120 may be pulled to open the arm 120 to at least a 45-degree angle relative to the center member 110. Depending on the position of the leaflet to be secured, the arm 120 may be opened to a larger degree, such as 90 degrees or up to 180 degrees in the fully open position. Once the first leaflet 19 is positioned adjacent the center member 110, the ends of a first wire 130 connected to the first arm 120 may be released, allowing the first arm 120 to return to the first position, capturing the first leaflet 19 between the first portion 122 of the arm 120 and the center member 110. The wire 130 connected to the second arm 120 may then be pulled to open the second arm 120 to at least 45 degrees, or up to 90 to 180 degrees. The fixation device 100 may then be moved such that the second leaflet 19 is adjacent the center member 110. The wire 130 connected to the second arm 120 may then be released, allowing the second arm to return to the first position, capturing the second leaflet 19 between the first portion 122 of the second arm 120 and the center member 110. If ultrasound imaging shows the position of the fixation device 100 needs to be adjusted, the wires 130 can be pulled to open one or both of the arms 120, thereby releasing the valve leaflets 19 and allowing readjustment. If the fixation device 100 is to be removed, the wires 130 may be pulled to move both arms 120 into the second position, as shown in FIG. 5, to release both leaflets 19 for removal of the fixation device 100. When the fixation device 100 is in the desired position with both valve leaflets 19 secured by the two arms 120, one end of each wire 130 may be pulled to remove the wires from the fixation device 100 and through the catheter 140. The catheter 140 may then be removed, by unscrewing the threaded connection 113. The resulting implanted fixation device 100 is shown in FIG. 6. The opening to the lumen in the first end 112 of the center member 110 previously holding the wires 130 may be filled with a plug to avoid having another conduit for blood flow extending through the device. The fixation device 100 may be a permanent implant, and may include a tissue ingrowth promoting material such as a polyester mesh covering the center member 110 and the arms 120. In some examples, the mesh may include tissue growth promoting substances.

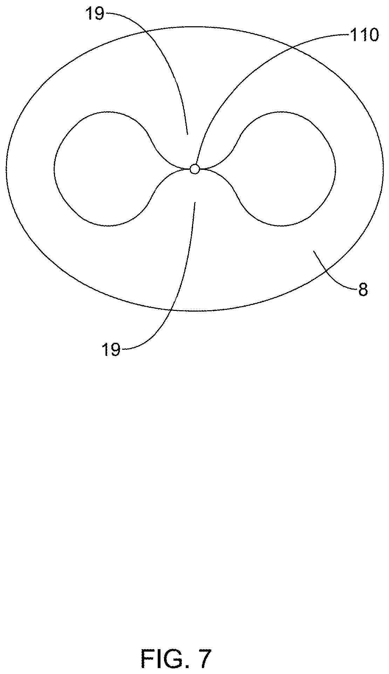

The arms 120 of the fixation device 100 are biased in the first, closed position with a force sufficient for the first portion 122 of each arm 120 to hold valve leaflets 19 securely between the arms 120 and the center member 110 during opening and closing of the valve as the heart beats, as shown in FIG. 6. The fixation device 100 may be placed in the center region of each leaflet 19 so that when the valve 18 opens, the portions of the valve leaflets 19 not held by the fixation device 100 may open to allow passage of blood, as shown in FIG 7.

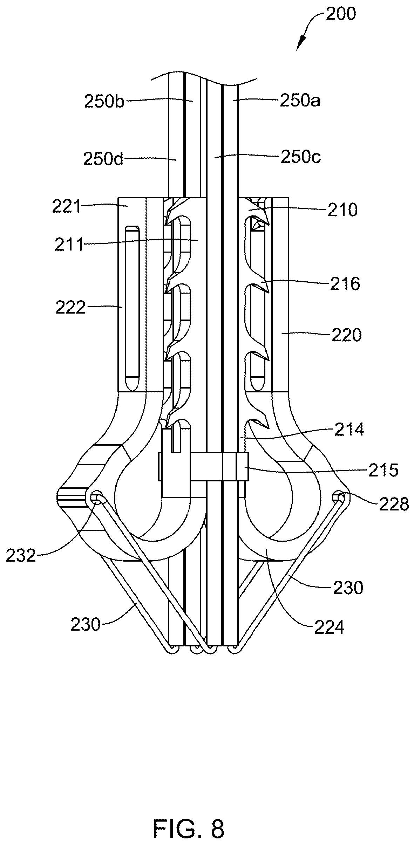

In another embodiment, the edge-to-edge fixation device 200 may include push rods 250 combined with cables or wires 230 to actuate the arms 220, as shown in FIGS. 8-10. Specifically, FIG. 8 illustrates a rod actuated fixation device 200 that includes a center member 210, first and second arms 220, and first and second actuators. In the example shown in FIG. 8, the actuators include a plurality of rods 250a, 250b, 250c, 250d (collectively 250) slidably connected to the center member 210, and first and second cables or wires 230. The center member 210 may have a connection element for removable engagement with a catheter, such as the threaded engagement described above with reference to the fixation device 100. The center member 210 may have a rod retainer 215 on both the front face 211 and rear face, through which the rods 250 may slide. In some embodiments, the front and rear rods may be combined into a single rod with two lumens, one to accept the front portion of the cable or wire and one to accept the rear portion of the cable or wire (not shown). The rods may be disposed through a lumen in the center member 210.

Each of the first and second arms 220 extends from the second end 214 of the center member 210 and have a free end 221. The center member 210 may include a surface texture configured to retain the valve leaflets when the fixation device 200 is engaged with opposing valve leaflets. In some examples, the surface texture includes a plurality of radially extending projections 216. The projections 216 may be blunt, tapered, or sharpened. In other examples, the surface of the center member 210 may be roughened or textured.

In some examples, the center member 210 and the first and second arms 220 are formed as a monolithic structure. In other examples, the first and second arms 220 may be formed separately and attached to the center member 210. The arms 220 may be made of a material that is more flexible than the center member 210.

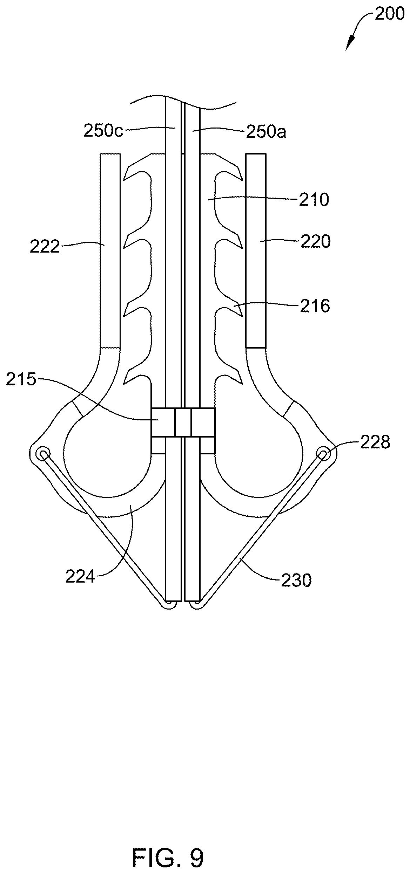

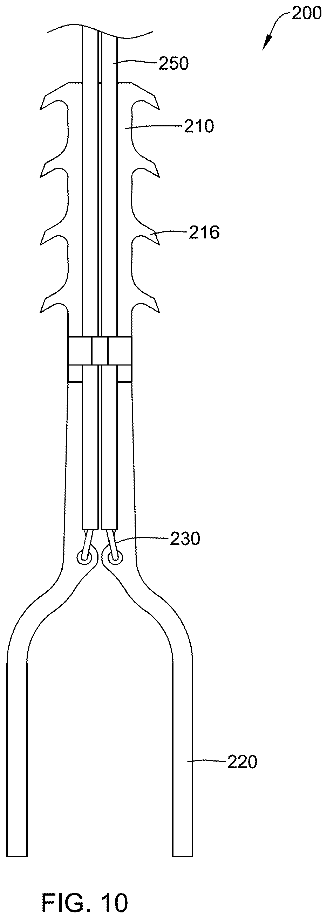

In the example shown in FIGS. 8 and 9, each arm 220 has a front rod 250a, 250c and a rear rod 250b, 250d slidingly associated with it. In some examples, each rod 250 has a lumen and a first wire 230 extends through one front rod 250a, 250c, through an opening 228 in the arm 220, forming a loop 232, and through one rear rod 250b, 250d. The wires 230 may extend through the delivery catheter (not shown) such that free ends of each wire 230 reside proximal of the catheter, outside the body. The arms 220 may have identical flexibility profiles as the arms 120 described above, being configured to move between a first, closed position as shown in FIGS. 8 and 9, and a second, fully open position as shown in FIG. 10. In the first position, at least a first portion 222 of each of the arms 220 is closely adjacent the projections 216. In some examples, the first portion 222 of each arm 220 may be in direct contact with the projections 216. Each arm 220 may have the same flexibility along its entire length. In other examples, the first portion 222 of each arm 220 may be rigid, and the second portion 224 of each arm 220 may be flexible, allowing the arms 220 to be moved into the second position. The arms 220 are biased in the first position.

In some examples, the wires 230 may be removably coupled to the rods 250 and the arms 220 may be actuated into the second position by pushing the rods 250 downward, which will translate into a downward force from the wire loop 232, pulling the arms 220 down into the second position. The wires 230 may be removably coupled to proximal ends of the rods 250 such that the wires 230 may be easily uncoupled and removed from the rods 250 once the fixation device 200 is in place. After the wires 230 have been removed from the rods 250, the rods may be pulled proximally out of the rod retainer 215.

In other examples, the wires 230 may slide through the rods 250 and the arms 220 may be actuated into the second position by pulling on the ends of the wires 230 while the rods 250 are held in place. The upward pulling force on the ends of the wires 230, will translate into a downward force as the loops 232 engage the openings 228 in the arms 220.

In a further example, the wires 230 may be replaced by a rigid linkage connected by pivot connections to the bottom ends of the rods 250 and to the arms 220 at the position of the opening 228 shown in FIG. 9. The pivot connection of the rigid linkage to the arms 220 at the position of the opening 228 may be removable, allowing the rigid linkage and associated rod 250 to be removed. In other examples, the connection may be permanent, and the lower portion of the rods 250 and linkage may be a permanent part of the fixation device 200, with just the upper portion of the rods 250 being removable at a quick-connect connection at the top of the center member 210.

In any of the above configurations, the arms 220 will move through a range of positions and into the second, fully open position, as shown in FIG. 10. The arms 220 may be actuated together by pulling on all four free ends of the two wires 230 and/or rods 250. Each arm 220 may also be actuated separately by pulling on the two free ends of the wire 230 and/or rods 250 connected to that arm.

In some examples, the rods 250 and wires 230 may be removed from the fixation device 200 after implantation. The wires 230 may be removed by pulling on one end and allowing the wire to be threaded out of the rods. The rods 250 may be pulled upward, sliding out of the rod retainer 215. In some examples, the portion of the rods 250 in contact with the center member 210 may be fixed to the center member 110 and the rods 250 may have a quick-connect coupling at the top end of the center member 210, such as a threaded connection. The upper portion of the rods 250 may be removed by unscrewing, leaving the lower portion of the rods 250 connected to the center member 210.

The fixation device 200 may be inserted through the vasculature and into the heart in the same manner as described above with reference to the fixation device 100, in either the first or second position, or any position therebetween. Once positioned within the valve to be treated, the fixation device 200 may be secured to both valve leaflets 19 at the same time or the leaflets 19 may be secured one at a time. Securing the leaflets one at a time may be easier, particularly when the heart is beating and the leaflets are moving. The flexibility of the fixation device 200 being secured to the leaflets at the same time or one at a time provides the advantage of allowing the operator to choose the implantation process that is desired for the particular patient.

Once the fixation device 200, in the first position, is disposed within the valve, the wire 230 and/or rod 250 on a first arm 220 may be pulled to open the arm 220 to at least a 45-degree angle relative to the center member 210. Depending on the position of the leaflet to be secured, the arm 220 may be opened to a larger degree, such as 90 degrees or up to 180 degrees in the fully open position. Once the leaflet is positioned adjacent the center member 210, the ends of a first wire 230 and/or rods 250 connected to a first arm 220 may be released, allowing the first arm 220 to return to the first position, capturing the first leaflet between the first portion 222 of the arm 220 and the center member 210. The wire 230 and/or rods 250 connected to the second arm 220 may then be pulled to open the second arm 220 to at least 45 degrees, or up to 90 to 180 degrees. The fixation device 200 may then be moved such that the second leaflet 19 is adjacent the center member 210. The wire 230 and/or rods 250 connected to the second arm 220 may then be released, allowing the second arm to return to the first position, capturing the second leaflet 19 between the first portion 222 of the second arm 220 and the center member 210. If ultrasound imaging shows the position of the fixation device 200 needs to be adjusted, one or both of the arms 220 may be opened, thereby releasing the valve leaflets 19 and allowing readjustment. If the fixation device 200 is to be removed, the wires 230 and/or rods 250 are pulled to move the arms 220 into the second position, as shown in FIG. 10. When the fixation device 200 is in the desired position with both valve leaflets 19 secured by the two arms 220, the wires 230 and rods 250 may be removed or retained as described above. The resulting implanted fixation device 200 is in the first position with the arms 220 biased in the first, closed position with a force sufficient for the first portion 222 of each arm 220 to hold valve leaflets 19 securely between the arms 220 and the center member 210 during opening and closing of the valve as the heart beats, as shown in FIG. 6. The free ends of the projections 216 may be blunt, tapered or sharpened, to aid in securing the fixation device 200 to the valve leaflets 19.

The fixation device 200 may be a permanent implant, and may include a tissue ingrowth promoting material such as a polyester mesh covering the center member 210 and the arms 220. In some examples, the mesh may include tissue growth promoting substances.

The materials that can be used for the various components of the fixation device 100, 200 for securing opposite heart valve leaflets (and/or other systems or components disclosed herein) and the various elements thereof disclosed herein may include those commonly associated with medical devices. For simplicity purposes, the following discussion makes reference to the fixation device 100 (and variations, systems or components disclosed herein). However, this is not intended to limit the devices and methods described herein, as the discussion may be applied to other elements, members, components, or devices disclosed herein.

In some embodiments, the embolic fixation device 100 (and variations, systems or components thereof disclosed herein) may be made from a metal, metal alloy, ceramics, zirconia, polymer (some examples of which are disclosed below), a metal-polymer composite, combinations thereof, and the like, or other suitable material. Some examples of suitable metals and metal alloys include stainless steel, such as 444V, 444L, and 314LV stainless steel; mild steel; nickel-titanium alloy such as linear-elastic and/or super-elastic nitinol; cobalt chromium alloys, titanium and its alloys, alumina, metals with diamond-like coatings (DLC) or titanium nitride coatings, other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R44035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY^{®}, PHYNOX^{®}, and the like); platinum enriched stainless steel; titanium; platinum; palladium; gold; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super-elastic varieties, may exhibit distinct and useful mechanical properties. Linear elastic and/or non-super-elastic nitinol may be distinguished from super-elastic nitinol in that the linear elastic and/or non-super-elastic nitinol does not display a substantial "super-elastic plateau" or "flag region" in its stress/strain curve like super-elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super-elastic plateau and/or flag region that may be seen with super-elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super-elastic nitinol in that linear elastic and/or non-super-elastic nitinol may accept up to about 2-5% strain while remaining substantially elastic (e.g., before plastically deforming) whereas super-elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60° Celsius (°C) to about 120°C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. For example, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM^{™} (available from Neo-Metrics) and GUM METAL^{™} (available from Toyota). In some other embodiments, a super-elastic alloy, for example a super-elastic nitinol can be used to achieve desired properties.

In at least some embodiments, portions or all of the fixation device 100 (and variations, systems or components thereof disclosed herein) may also be doped with, made of, or otherwise include a radiopaque material. Radiopaque materials are understood to be materials capable of producing a relatively bright image on a fluoroscopy screen or another imaging technique during a medical procedure. This relatively bright image aids a user in determining the location of the fixation device 100 (and variations, systems or components thereof disclosed herein). Some examples of radiopaque materials can include, but are not limited to, gold, platinum, palladium, tantalum, tungsten alloy, polymer material loaded with a radiopaque filler, and the like. Additionally, other radiopaque marker bands and/or coils may also be incorporated into the design of the fixation device 100 (and variations, systems or components thereof disclosed herein) to achieve the same result.

In some embodiments, the fixation device 100 (and variations, systems or components thereof disclosed herein) and/or portions thereof, may be made from or include a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex^{®} high-density polyethylene, Marlex^{®} low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, polyurethane silicone copolymers (for example, Elast-Eon^{®} from AorTech Biomaterials or ChronoSil^{®} from AdvanSource Biomaterials), biocompatible polymers, other suitable materials, or mixtures, combinations, copolymers thereof, polymer/metal composites, and the like. In some embodiments, the sheath can be blended with a liquid crystal polymer (LCP). For example, the mixture can contain up to about 6 percent LCP.

In some embodiments, the fixation device 100 (and variations, systems or components thereof disclosed herein) may include and/or be treated with a suitable therapeutic agent. Some examples of suitable therapeutic agents may include anti-thrombogenic agents (such as heparin, heparin derivatives, urokinase, and PPack (dextrophenylalanine proline arginine chloromethylketone)); anti-proliferative agents (such as enoxaparin, angiopeptin, monoclonal antibodies capable of blocking smooth muscle cell proliferation, hirudin, and acetylsalicylic acid); anti-inflammatory agents (such as dexamethasone, prednisolone, corticosterone, budesonide, estrogen, sulfasalazine, and mesalamine); antineoplastic/antiproliferative/anti-mitotic agents (such as paclitaxel, 5-fluorouracil, cisplatin, vinblastine, vincristine, epothilones, endostatin, angiostatin and thymidine kinase inhibitors); anesthetic agents (such as lidocaine, bupivacaine, and ropivacaine); anti-coagulants (such as D-Phe-Pro-Arg chloromethyl ketone, an RGD peptide-containing compound, heparin, anti-thrombin compounds, platelet receptor antagonists, anti-thrombin antibodies, anti-platelet receptor antibodies, aspirin, prostaglandin inhibitors, platelet inhibitors, and tick antiplatelet peptides); vascular cell growth promoters (such as growth factor inhibitors, growth factor receptor antagonists, transcriptional activators, and translational promoters); vascular cell growth inhibitors (such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin); cholesterol-lowering agents; vasodilating agents; and agents which interfere with endogenous vasoactive mechanisms.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. A fixation device (100; 200) for engaging heart valve leaflets, comprising:
a center member (110; 210) with a first end (112), an opposite second end (114; 214), and a length extending therebetween;
first and second arms (120; 220) each having a first free end (121; 221) and an opposite second end, the second end of each arm (120; 220) extending from the second end (114; 214) of the center member (110; 210), the first and second arms biased in a first position adjacent the center member (110; 210) and moveable to a second position spaced apart from the center member (110; 210);
wherein the fixation device is configured to capture a first leaflet (19) between a first portion (122) of the first arm (120) and the center member (110) and a second leaflet (19) between a first portion (122) of the second arm (220) and the center member (110) when returning from the second position to the first position;
an actuator configured to move the first and second arms (120; 220) from the first position to the second position;
wherein the actuator is removable and includes a first wire (130; 230) removably connected to the first arm (120; 220) and a second wire (130; 230) removably connected to the second arm (120; 220);
**characterized in that** the first arm (120; 220) has an opening (128) configured to receive a loop (132) in the first wire (130; 230) and the second arm (120; 220) has an opening (128) configured to receive a loop (123) in the second wire (130; 230);
wherein opposing free ends of each wire (130; 230) extend through a lumen in the center member (110; 210), wherein a pulling force applied on the free ends moves the first and second arms (120; 220) from the biased first position to the second position, wherein releasing the pulling force allows the first and second arms (120; 220) to return to the first position.

2. The fixation device of claim 1, wherein the center member (110; 210) includes a plurality of radially extending projections (116; 216).

3. The fixation device of claim 2, wherein when in the first position, at least the first portion of each of the first and second arms (120; 220) is in contact with the projections (116; 216).

4. The fixation device of any one of claims 1-3, wherein the first end of the center member (110; 210) includes a connection element (113) configured to be removably connected to a catheter shaft (140).

5. The fixation device of any one of claims 1-4, wherein the first and second arms (120; 220) are independently actuatable.

6. The fixation device of claim 1, wherein the actuator includes first front and rear rods (250) removably connected to the first arm (220) via the first wire (230), and second front and rear rods (250) removably connected to the second arm (220) via the second wire (230).

7. The fixation device of claim 6, wherein opposing free ends of the first wire (230) extend through lumens in the first front and rear rods (250) and opposing free ends of the second wire (230) extend through lumens in the second front and rear rods (250).

8. The fixation device of claim 7, wherein a pulling force applied on the opposing free ends of the first wire (230) moves the first arm (220) from the biased first position to the second position, and a pulling force applied on the opposing free ends of the second wire (230) moves the second arm (220) from the biased first position to the second position, wherein releasing the pulling force allows the first or second arm (220) to return to the first position, wherein the first and second arms (220) are configured to be actuated together or independently.

9. The fixation member of claim 8, wherein the first wire (230) is slidably disposed within the first front and rear rods (250) and the second wire (230) is slidably disposed within the second front and rear rods (250) such that a pulling force on a single free end of the first and second wires (230) removes the wires (230) from the front and rear rod (250) and arm (220) to which it is connected.

10. The fixation member of claim 7, wherein the first wire (230) is removably coupled to the first front and rear rods (250) and the second wire (230) is removably coupled to the second front and rear rods (250), wherein pushing the first front and rear rods (250) downward moves the first arm (220) from the first to the second position and pushing the second front and rear rods moves the second arm (220) from the first to the second position.

11. The fixation member of claim 10, wherein the removably coupled engagement between the first wire (230) and the first front and rear rods (250) and the removably coupled engagement between the second wire (230) and the second front and rear rods (250) allows the first and second wires (230) to be uncoupled and pulled proximally from the first and second front and rear rods (250).

## Patentansprüche

1. Befestigungsvorrichtung (100; 200) zum Ineingriffnehmen von Herzklappensegeln, umfassend:
ein zentrales Element (110; 210) mit einem ersten Ende (112), einem gegenüberliegenden zweiten Ende (114; 214) und einer sich dazwischen erstreckenden Länge;
einen ersten und einen zweiten Arm (120; 220), die jeweils ein erstes freies Ende (121; 221) und ein gegenüberliegendes zweites Ende haben, wobei sich das zweite Ende jedes Arms (120; 220) von dem zweiten Ende (114; 214) des zentralen Elements (110; 210) erstreckt, der erste und der zweite Arm in einer ersten Position neben dem zentralen Element (110; 210) vorgespannt und in eine vom zentralen Element (110; 210) beabstandete zweite Position beweglich sind;
wobei die Befestigungsvorrichtung dazu ausgelegt ist, ein erstes Segel (19) zwischen einem ersten Abschnitt (122) des ersten Arms (120) und dem zentralen Element (110) und ein zweites Segel (19) zwischen einem ersten Abschnitt (122) des zweiten Arms (220) und dem zentralen Element (110) zu erfassen, wenn er aus der zweiten Position in die erste Position zurückkehrt;
eine Betätigungseinrichtung, die dazu ausgelegt ist, den ersten und den zweiten Arm (120; 220) aus der ersten Position in die zweite Position zu bewegen;
wobei die Betätigungseinrichtung entfernbar ist und einen ersten Draht (130; 230), der entfernbar mit dem ersten Arm (120; 220) verbunden ist, und einen zweiten Draht (130; 230), der entfernbar mit dem zweiten Arm (120; 220) verbunden ist, aufweist;
**dadurch gekennzeichnet, dass** der erste Arm (120; 220) eine Öffnung (128) hat, die dazu ausgelegt ist, eine Schlaufe (132) in dem ersten Draht (130; 230) aufzunehmen, und der zweite Arm (120; 220) eine Öffnung (128) hat, die dazu ausgelegt ist, eine Schlaufe (123) in dem zweiten Draht (130; 230) aufzunehmen;
wobei sich gegenüberliegende freie Enden eines jeden Drahtes (130; 230) durch ein Lumen in dem zentralen Element (110; 210) erstrecken, wobei eine Zugkraft, die auf die freien Enden aufgebracht wird, den ersten und den zweiten Arm (120; 220) aus der vorgespannten ersten Position in die zweite Position bewegt, wobei das Freigeben der Zugkraft dem erstem und dem zweiten Arm (120; 220) erlaubt, in die erste Position zurückzukehren.

2. Befestigungsvorrichtung nach Anspruch 1, wobei das zentrale Element (110; 210) eine Vielzahl von sich radial erstreckenden Vorsprüngen (116; 216) aufweist.

3. Befestigungsvorrichtung nach Anspruch 2, wobei, wenn in der ersten Position, mindestens der erste Abschnitt eines jeden des ersten und des zweiten Arms (120; 220) mit den Vorsprüngen (116; 216) in Kontakt ist.

4. Befestigungsvorrichtung nach einem der Ansprüche 1-3, wobei das erste Ende des zentralen Elements (110; 210) ein Verbindungselement (113) aufweist, das dazu ausgelegt ist, entfernbar mit einem Katheterschaft (140) verbunden zu werden.

5. Befestigungsvorrichtung nach einem der Ansprüche 1-4, wobei der erste und der zweite Arm (120; 220) unabhängig betätigbar sind.

6. Befestigungsvorrichtung nach Anspruch 1, wobei die Betätigungseinrichtung eine erste vordere und eine erste hintere Stange (250) aufweist, die entfernbar mit dem ersten Arm (220) über den ersten Draht (230) verbunden sind, und eine zweite vordere und eine zweite hintere Stange (250), die entfernbar über den zweiten Draht (230) mit dem zweiten Arm (220) verbunden sind.

7. Befestigungsvorrichtung nach Anspruch 6, wobei sich die gegenüberliegenden freien Enden des ersten Drahtes (230) durch Lumen in der ersten vorderen und der ersten hinteren Stange (250) erstrecken und sich die gegenüberliegenden freien Enden des zweiten Drahtes (230) durch Lumen in der zweiten vorderen und der zweiten hinteren Stange (250) erstrecken.

8. Befestigungsvorrichtung nach Anspruch 7, wobei eine Zugkraft, die auf die gegenüberliegenden freien Enden des ersten Drahtes (230) aufgebracht wird, den ersten Arm (220) aus der vorgespannten ersten Position in die zweite Position bewegt, und eine Zugkraft, die auf die gegenüberliegenden freien Enden des zweiten Drahtes (230) aufgebracht wird, den zweiten Arm (220) aus der vorgespannten ersten Position in die zweite Position bewegt, wobei das Freigeben der Zugkraft dem ersten oder dem zweiten Arm (220) erlaubt, in die erste Position zurückzukehren, wobei der erste und der zweite Arm (220) dazu ausgelegt sind, zusammen oder unabhängig betätigt zu werden.

9. Befestigungsvorrichtung nach Anspruch 8, wobei der erste Draht (230) verschiebbar innerhalb der ersten vorderen und der ersten hinteren Stange (250) angeordnet ist und der zweite Draht (230) verschiebbar innerhalb der zweiten vorderen und der zweiten hinteren Stange (250) angeordnet ist, so dass eine Zugkraft auf einem einzelnen freien Ende des ersten und des zweiten Drahtes (230) die Drähte (230) aus der vorderen und der hinteren Stange (250) und dem Arm (220), mit dem sie verbunden sind, entfernt.

10. Befestigungsvorrichtung nach Anspruch 7, wobei der erste Draht (230) entfernbar mit der ersten vorderen und der ersten hinteren Stange (250) gekoppelt ist und der zweite Draht (230) entfernbar mit der zweiten vorderen und der zweiten hinteren Stange (250) gekoppelt ist, wobei das Herunterdrücken der ersten vorderen und der ersten hinteren Stange (250) den ersten Arm (220) aus der ersten in die zweite Position bewegt und das Herunterdrücken der zweiten vorderen und der zweiten hinteren Stange den zweiten Arm (220) aus der ersten in die zweite Position bewegt.

11. Befestigungsvorrichtung nach Anspruch 10, wobei der entfernbar gekoppelte Eingriff zwischen dem ersten Draht (230) und der ersten vorderen und der ersten hinteren Stange (250) und der entfernbar gekoppelte Eingriff zwischen dem zweiten Draht (230) und der zweiten vorderen und der zweiten hinteren Stange (250) ermöglicht, dass der erste und der zweite Draht (230) entkoppelt und proximal aus der ersten und der zweiten hinteren Stange (250) gezogen werden.

## Revendications

1. Dispositif de fixation (100 ; 200) pour mettre en prise des feuillets de valvule cardiaque, comprenant :
un élément central (110 ; 210) avec une première extrémité (112), une seconde extrémité (114 ; 214) opposée, et une longueur s'étendant entre celles-ci ;
des premier et second bras (120 ; 220) ayant chacun une première extrémité libre (121 ; 221) et une seconde extrémité opposée, la seconde extrémité de chaque bras (120 ; 220) s'étendant à partir de la seconde extrémité (114 ; 214) de l'élément central (110 ; 210), les premier et second bras étant sollicités dans une première position adjacente à l'élément central (110 ; 210) et pouvant être déplacés vers une seconde position espacée de l'élément central (110 ; 210) ;
dans lequel le dispositif de fixation est configuré pour capturer un premier feuillet (19) entre une première partie (122) du premier bras (120) et l'élément central (110) et un second feuillet (19) entre une première partie (122) du second bras (220) et l'élément central (110) lors du retour de la seconde position à la première position ;
un actionneur configuré pour déplacer les premier et second bras (120 ; 220) de la première position à la seconde position ;
dans lequel l'actionneur est amovible et comporte un premier fil (130 ; 230) relié de manière amovible au premier bras (120 ; 220) et un second fil (130 ; 230) relié de manière amovible au second bras (120 ; 220) ;
**caractérisé en ce que** le premier bras (120 ; 220) présente une ouverture (128) configurée pour recevoir une boucle (132) dans le premier fil (130 ; 230) et le second bras (120 ; 220) présente une ouverture (128) configurée pour recevoir une boucle (123) dans le second fil (130 ; 230) ;
dans lequel les extrémités libres opposées de chaque fil (130 ; 230) s'étendent à travers une lumière dans l'élément central (110 ; 210), dans lequel une force de traction appliquée sur les extrémités libres déplace les premier et second bras (120 ; 220) de la première position sollicitée à la seconde position, dans lequel la libération de la force de traction permet aux premier et second bras (120 ; 220) de revenir à la première position.

2. Dispositif de fixation selon la revendication 1, dans lequel l'élément central (110; 210) comporte une pluralité de protubérances s'étendant radialement (116 ; 216).

3. Dispositif de fixation selon la revendication 2, dans lequel, lorsqu'elle est dans la première position, au moins la première partie de chacun des premier et second bras (120 ; 220) est en contact avec les protubérances (116 ; 216).

4. Dispositif de fixation selon l'une quelconque des revendications 1 à 3, dans lequel la première extrémité de l'élément central (110 ; 210) comporte un élément de liaison (113) configuré pour être relié de manière amovible à une tige de cathéter (140).

5. Dispositif de fixation selon l'une quelconque des revendications 1 à 4, dans lequel les premier et second bras (120 ; 220) peuvent être actionnés indépendamment.

6. Dispositif de fixation selon la revendication 1, dans lequel l'actionneur comporte des premières tiges avant et arrière (250) reliées de manière amovible au premier bras (220) par le biais du premier fil (230), et des secondes tiges avant et arrière (250) reliées de manière amovible au second bras (220) par le biais du second fil (230).

7. Dispositif de fixation selon la revendication 6, dans lequel des extrémités libres opposées du premier fil (230) s'étendent à travers des lumières dans les premières tiges avant et arrière (250) et des extrémités libres opposées du second fil (230) s'étendent à travers des lumières dans les secondes tiges avant et arrière (250).

8. Dispositif de fixation selon la revendication 7, dans lequel une force de traction appliquée sur les extrémités libres opposées du premier fil (230) déplace le premier bras (220) de la première position sollicitée à la seconde position, et une force de traction appliquée sur les extrémités libres opposées du second fil (230) déplace le second bras (220) de la première position sollicitée à la seconde position, la libération de la force de traction permettant au premier ou second bras (220) de revenir à la première position, les premier et second bras (220) étant configurés pour être actionnés ensemble ou indépendamment.

9. Élément de fixation selon la revendication 8, dans lequel le premier fil (230) est disposé de manière coulissante à l'intérieur des premières tiges avant et arrière (250) et le second fil (230) est disposé de manière coulissante à l'intérieur des secondes tiges avant et arrière (250) de telle sorte qu'une force de traction sur une seule extrémité libre des premier et second fils (230) retire les fils (230) des tiges avant et arrière (250) et du bras (220) auxquels ils sont reliés.

10. Élément de fixation selon la revendication 7, dans lequel le premier fil (230) est accouplé de manière amovible aux premières tiges avant et arrière (250) et le second fil (230) est accouplé de manière amovible aux secondes tiges avant et arrière (250), dans lequel pousser les premières tiges avant et arrière (250) déplace le premier bras (220) vers le bas de la première à la seconde position et pousser les secondes tiges avant et arrière déplace le second bras (220) de la première à la seconde position.

11. Élément de fixation selon la revendication 10, dans lequel la prise accouplée de manière amovible entre le premier fil (230) et les premières tiges avant et arrière (250) et la prise accouplée de manière amovible entre le second fil (230) et les secondes tiges avant et arrière (250) permettent aux premier et second fils (230) d'être découplés et tirés de manière proximale à partir des première et seconde tiges avant et arrière (250).
